# EUROPEAN PATENT APPLICATION

(11) **EP 1 921 466 A2**
(43) Date of publication of application: **14.05.2008**
(21) Application number: 07119570.5
(22) Date of filing: 30.10.2007
(51) Int. Cl.: G01T 1/00

(54) **Radiation image radiographing system and radiation image detecting apparatus**

(30) Priority: 08.11.2006 JP 2006302601
(71) Applicant: Konica Minolta Medical & Graphic, Inc., Hino-shi, Tokyo 191-8511 (JP)
(72) Inventor: Amitani, Kouji c/o Konica Minolta Medical & Graphic, Inc., Tokyo 191-8511 (JP)
(74) Representative: Gille Hrabal Struck Neidlein Prop Roos

(57) **Abstract**

The console 7, when the reception of the radiation image data transmitted from the radiation image detecting apparatus 6 is completed (Yes at Step S8), transmits the reception success signal to the radiation image detecting apparatus 6 (Step S9). The radiation image detecting apparatus 6, upon receipt of the reception success signal from the console 7 (Yes at Step S11), turns on the transmission button 64 in blue (Step S12) to display completion of the data transmission. The operator confirms that the transmission button 64 is turned on in blue and presses the deletion button 65. The radiation image detecting apparatus 6, upon receipt of input of the deletion button 65, deletes the radiation image data stored in the image storing section 66 (Step S13).

## Description

This application is based on Japanese Patent Application No. 2006-302601 filed on Nov. 8, 2006 in Japanese Patent Office, the entire content of which is hereby incorporated by reference.

### BACKGROUND OF THE INVENTION

The present invention relates to a radiation image detecting apparatus for detecting radiation represented by X-rays, thereby generating radiation image data and a radiation image radiographing system using the radiation image detecting apparatus.

At the field of medical analysis, a computed radiography (CR) system capable of handling a radiation image as digital data is put into practical use as a radiation image detecting apparatus. Furthermore, to respond to use thereof in a medical facility having a plurality of radiographing rooms, introduction of a large-scale CR system including a plurality of reading apparatuses and a plurality of control devices (consoles) which are connected with a network is proposed (refer to Patent Document 1). According to the large-scale CR system of Patent Document 1, even if a CR cassette is read by any reading apparatus, the reading apparatus reads the cassette ID accompanying the CR cassette and identifies the control device (console) in which the CR cassette has been registered on the basis of the cassette ID, and radiation image data is transmitted to and displayed on the control device in which the CR cassette has been registered. Therefore, for example, even if radiography is executed many times extending over the plurality of the radiographing rooms, the radiation image data is displayed on a single control device, so that the operation efficiency can be improved.

And, in place of the CR cassette aforementioned, a flat panel detector (FPD) having a built-in radiation detection element arranged two-dimensionally on a substrate for outputting an electric signal according to the dosage of radiation emitted to the radiation detection element is proposed as a radiation image detecting apparatus. By use of the FPD, there is no need to install a reading apparatus for irradiating with exciting light and reading a radiation image, and radiation image data can be obtained directly, so that compared with the case using the CR cassette, the system itself can be made smaller and the radiographing operation can be performed smoothly.

Conventionally, though the FPD is installed in the radiographing room, a portable one has been required to realize quicker radiography in a wide range of body parts. Further, to improve more the radiographing degree of freedom, a portable FPD having a built-in battery for realizing wireless communication has been designed (refer to Patent Document 2). In Patent Document 2, from the radiation image data obtained by radiographing with the portable FPD, a subsampled image for preview is prepared and transferred to the control device by wireless communication, thus the radiographing conditions are confirmed. And, when it is judged by the control device that re-radiography is not required for all the radiation image data, the portable FPD is mounted on the cradle after end of the radiography and the radiation image data stored in the memory of the portable FPD is transferred to the control device by wired communication of cradle.

However, in the aforementioned constitution of Patent Document 2, to transmit the radiation image data to the control device, the FPD must be mounted on the cradle, so that a very complicated operation is forced to be performed, thus the immediate effect of the portable FPD cannot be used.

Further, in the radiography using the portable FPD, in consideration of that the number of radiographing images per patient is varied according to the disease and radiographing body part and the portable FPD will be adopted in place of the CR cassette used in the large-scale CR system aforementioned, it is desirable that the portable FPD can execute radiography continuously several times. On the other hand, in the constitution of Patent Document 2, when the number of continuous radiographing times is increased, the capacity of the built-in memory must be increased in correspondence to it, and the constitution is complicated, thus there is a fear of causing enlargement of the portable FPD.

Therefore, as a method for keeping the portable FPD small-sized and performing the radiographing operation smoothly, it is desirable to transmit the radiation image data by wireless communication. However, through the wireless communication, since the radiation image data is high-resolution lossless compression image data, the amount of data is large, the transmission cannot be completed in a short time, and it is difficult to maintain always stably the receivable state of the control device and therefore, a problem arises that defective communication occurs easily. And, when the radiation image data is transmitted to the control device, and the radiation image data is deleted from the portable FPD, and then an occurrence of defective communication is ascertained, the radiography must be executed again, and not only the operation is complicated but also a burden such as unnecessary exposure is imposed on a patient.
Patent Document 1: Japanese Unexamined Patent Application Publication 2002-159476
Patent Document 2: Japanese Unexamined Patent Application Publication 2002-248095

### SUMMARY

Therefore, the present invention was developed with the foregoing problem in view and is intended to provide a radiation image radiographing system and a radiation image detecting apparatus, when transmitting radiation image data by wireless communication, for realizing high efficiency of a radiographing operation by making a portable FPD compact and preventing an exposure amount of a patient from increasing unnecessarily.

To accomplish the above object, the radiation image radiographing system of the present invention is a radiation image radiographing system connected by a network including a portable radiation image detecting apparatus for detecting radiation having passed through a subject, thereby generating radiation image data, and being capable of transmitting the generated radiation image data and a console for registering radiographing order information relating to radiography and correlating the concerned radiographing order information with the radiation image detecting apparatus, wherein the console includes a first receiving section for receiving the radiation image data transmitted from the radiation image detecting apparatus by wireless communication and a first transmitting section, when the reception of the radiation image data succeeds, for transmitting a reception success signal to the radiation image detecting apparatus, and the radiation image detecting apparatus includes an image storing section for storing the generated radiation image data of at least one radiographing, a second transmission section for transmitting the radiation image data stored in the image storing section to the console by wireless communication, a second reception section for receiving the reception success signal transmitted from the first transmission section, and a deletion control device for executing control for deleting the radiation image data transmitted by the second transmission section from the image storing section when the second transmission section transmits the radiation image data and then the second receiving section receives the reception success signal.

Further, to accomplish the above object, the radiation image detecting apparatus of the present invention is a portable radiation image detecting apparatus capable of transmitting radiation image data generated by detecting radiation having passed through a subject to a console for registering radiographing order information relating to radiography, including an imaging panel for generating radiation image data by detecting the radiation having passed through the subject, an image storing section for storing radiation image data of at least one radiographing generated by the imaging panel, an image data transmission section for transmitting the radiation image data stored in the image storing section to the console by wireless communication, a success signal reception section for receiving a reception success signal indicating that the reception of the radiation image data by the console succeeds, and a deletion control device for executing control for deleting the radiation image data transmitted by the image data transmission section from the image storing section when the image data transmission section transmits the radiation image data and then the success signal receiving section receives the reception success signal.

### BRIEF DESCRIPTION OF THE DRAWINGS

Fig. 1 is a drawing showing a schematic constitution of an embodiment of the radiation image radiographing system relating to the present invention.
Fig. 2 is a block diagram showing the constitution of the essential section of the console.
Fig. 3 is a perspective view of the radiation image detecting apparatus.
Fig. 4 is an equivalent circuit diagram of the photoelectric conversion section composing the signal detection section.
Fig. 5 is an equivalent circuit diagram of the signal detection section having the photoelectric conversion sections arranged two-dimensionally.
Fig. 6 is a block diagram showing the constitution of the essential section of the radiation image detecting apparatus.
Fig. 7 is a flow chart for describing the operation of the radiation image radiographing system relating to the first embodiment.
Fig. 8 is a flow chart when the operation of the radiation image radiographing system relating to the first embodiment shown in Fig. 7 is partially modified.
Fig. 9 is a flow chart for describing the process for the alternative console of the radiation image detecting apparatus in the radiation image radiographing system.
Fig. 10 is a flow chart for describing the operation of the radiation image radiographing system relating to the second embodiment.

### DETAILED DESCRIPTION OF THE PREFERRED EMBODIMENTS

Hereinafter, the embodiments of the present invention will be described with reference to the accompanying drawings. The technical range of the present invention is not limited to the description of the embodiments.

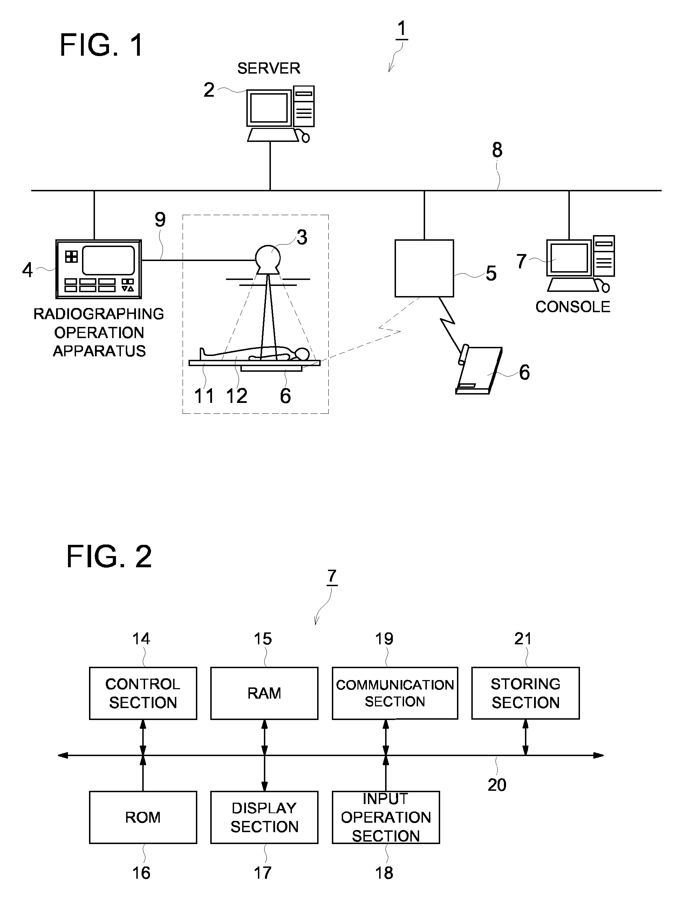

Fig. 1 is a drawing showing a schematic constitution of an embodiment of a radiation image radiographing system 1 relating to the present invention.

The radiation image radiographing system 1 of this embodiment, as shown in Fig. 1, includes a server 2 for managing information on the radiography, a radiographing operation apparatus 4 for performing an operation relating to the radiography, a base station 5 for communicating by a wireless communication system such as a radio LAN (local area network), and a console 7 for performing an image processing for radiation image data generated by a radiation image detecting apparatus 6 which are connected via a network 8.
To the radiographing operation apparatus 4, a radiation image radiographing apparatus 3 for irradiating a subject with radiation and executing radiography is connected via a cable 9. Here, the network 8 may be an exclusive communication circuit of the concerned system, however for the reason that the degree of freedom of the system configuration is lowered, an existing circuit such as Ethernet (registered trademark) is preferable. Further, although not drawn, a plurality of radiation image detecting apparatuses 6 and a plurality of consoles 7 are installed and a plurality of images can be captured extending over a plurality of imaging rooms.

The server 2 is composed of a computer and includes a control section for controlling the sections composing the server 2, an input operation section for inputting various information and a user's instruction, and an external storing section for storing various information (they are not drawn). The control section correlates patient information and radiographing information inputted from the input operation section with each other, thereby generates radiographing order information, and permits the external storing section to store the radiographing order information. Further, as described later, the control section receives the cassette ID which is transmitted from the console 7 and is assigned to the radiation image detecting apparatus 6, radiographing order information, and correlating information corresponding to the console ID assigned to the console 7 and stores those information in the external storing section. Here, the patient information is information relating to a patient 12 such as the name, age, distinction of sex, date of birth, and patient ID number for identifying the patient 12. Further, the radiographing information is information necessary to execute radiography such as the radiographing part (the part of the patient body to be radiographed) and radiographing direction and method.

Further, in the radiation image radiographing system 1 of this embodiment, the server 2 can select the transmission destination of radiation image data from the radiation image detecting apparatus 6 among a plurality of consoles or can select the transmission destination of a signal from the console 7 among a plurality of radiation image detecting apparatus 6. In other words, the server 2 is equivalent to a selection means of the present invention.

The radiation image radiographing apparatus 3 irradiates the patient 12 who is a subject loaded on a bed 11 with radiation and under the bed 11, a detecting apparatus mounting port (not drawn) for mounting the radiation image detecting apparatus 6 is installed. The radiation image radiographing apparatus 3 is controlled by the radiographing operation apparatus 4 and executes radiography under predetermined radiographing conditions.

The base station 5, when the radiation image detecting apparatus 6 and console 7 communicate by the wireless communication system such as the radio LAN, has a function for relaying the communication.

Fig. 2 is a block diagram showing the constitution of the essential section of the console 7.

The console 7, as shown in Fig. 2, is composed of a computer including a control section 14, a RAM 15, a ROM 16, a display section 17, an input operation section 18, a communication section 19, and a storing section 21 and the sections are connected to each other via a bus 20. Further, the console 7 is an apparatus integrally composing an image display section and an image processing apparatus.

The RAM (random access memory) 15, in various processes executed and controlled by the control section 14, forms a work area for temporarily storing various programs which are read from the ROM 16 and can be executed by the control section 14, input or output data, and parameters.

The ROM (read only memory) 16 is composed of a nonvolatile semiconductor memory and others and stores the control program executed by the control section 14 and image processing conditions.

The display section 17 is composed of, for example, a CRT (cathode ray tube) and an LCD (liquid crystal display) and, according to an instruction of a display signal which is outputted and inputted from the control section 14, displays various screens.

The input operation section 18 is composed of, for example, a keyboard and a mouse and outputs a pressing signal of the pressed key of the keyboard or an operation signal by the mouse to the control section 14 as an input signal. Concretely, with the input operation section 18, the patient information such as the cassette ID and patient name which are assigned to the radiation image detecting apparatus 6 can be inputted. Further, a constitution may be used so that a bar code reader is used as an input operation section 18, and the bar code assigned to the radiation image detecting apparatus 6 is read, thus the cassette ID is inputted.

The communication section 19, by the wireless communication system such as IEEE802.11b/g, Bluetooth, or UWB (ultra wide band), via the base station 5, communicates various information with the radiation image detecting apparatus 6. In this embodiment, the communication section 19 and control section 14 which will be described later can compose the first transmission section and first reception section of the present invention.

The control section 14 is composed of, for example, a CPU (central processing unit), reads a predetermined program stored in the ROM 16, loads it into the work area of the RAM 15, and executes various processes according to the concerned program. The control section 14 receives the radiographing order information stored in the external storing section of the server 2 via the network 8 and transmits the information about the radiographing part from the radiographing information included in the received radiographing order information to the radiation image detecting apparatus 6 by wireless communication via the base station 5. Further, the control section 14 receives radiation image data from the radiation image detecting apparatus 6 by wireless communication via the base station 5 and performs the image processes such as the normalization process and gradation process for the radiation image data on the basis of the image processing conditions stored in the ROM 16. Furthermore, the control section 14 correlates the radiographing order information aforementioned with the radiation image data and permits the storing section 21 to store it. Further, the control section 14 is equivalent to the correlating device of the present invention.

The storing section 21 stores the radiographing order information which is transmitted from the server 2 and is received via the communication section 19 and the radiation image data transmitted from the radiation image detecting apparatus 6, and correlates the radiographing order information with the radiation image data, and then stores them.

The radiation image detecting apparatus 6 detects radiation which is emitted from the radiation image radiographing apparatus 3 and transmits the patient 12, thereby obtains the radiation image data and it is a portable FPD composed of an imaging panel called a flat panel detector (FPD) stored in a cassette.

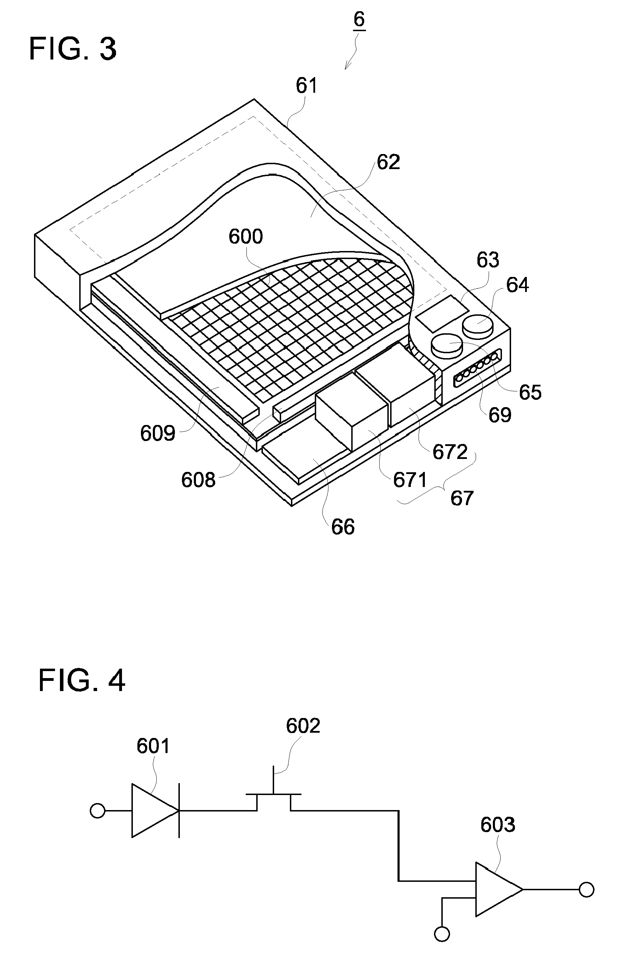

Hereinafter, the structure of the radiation image detecting apparatus 6 will be described by referring to Figs. 3 to 5. Fig. 3 is a perspective view of the radiation image detecting apparatus 6. As shown in Fig. 3, the radiation image detecting apparatus 6 has a frame 61 for protecting the inside thereof and is structured so as to be carried as a cassette.

Inside the frame 61, an imaging panel 62 for converting emitted radiation to an electric signal is formed in layers. On the radiation irradiation side of the imaging panels 62, a light emission layer (not illustrated) for emitting light according to the intensity of the incident radiation is installed.

The light emission layer is generally called a scintillator layer, which is, for example, mainly composed of a fluorescent substance and on the basis of the incident radiation, outputs electromagnetic waves with a wavelength between 300 nm and 800 nm, that is, electromagnetic waves (light) extending over from ultraviolet rays to infrared rays including visible rays in the middle.

The fluorescent substance used in the light emission layer can use, for example, a one composed of CaWO₄ as a base substance or a one in which a light emission center substance is activated in the base substance such as CsI:Tl, Gd₂O₂S:Tb, or ZnS:Ag. Further, assuming the rare-earth element as M, a fluorescent substance expressed by a general formula of (Gd, M, Eu)₂O₃ can be used. Particularly, CsI:Tl and Gd₂O₂S:Tb are preferable because the radiation absorption rate and light emission efficiency thereof are high and by use of them, a high-quality image with low noise can be obtained.

On the side of the light emission layer to which the radiation is emitted and the opposite side thereof, a signal detection section 600 including photoelectric conversion sections arranged in a matrix shape for converting electromagnetic waves (light) outputted from the light emission layer to electric energy, for storing it, and for outputting an image signal based on the stored electric energy is formed. Further, a signal outputted from one photoelectric conversion section is a signal equivalent to one pixel which is a minimum unit composing radiation image data.

Here, the circuit constitution of the imaging panel 62 will be described. Fig. 4 is an equivalent circuit diagram of the photoelectric conversion section composing the signal detection section 600.

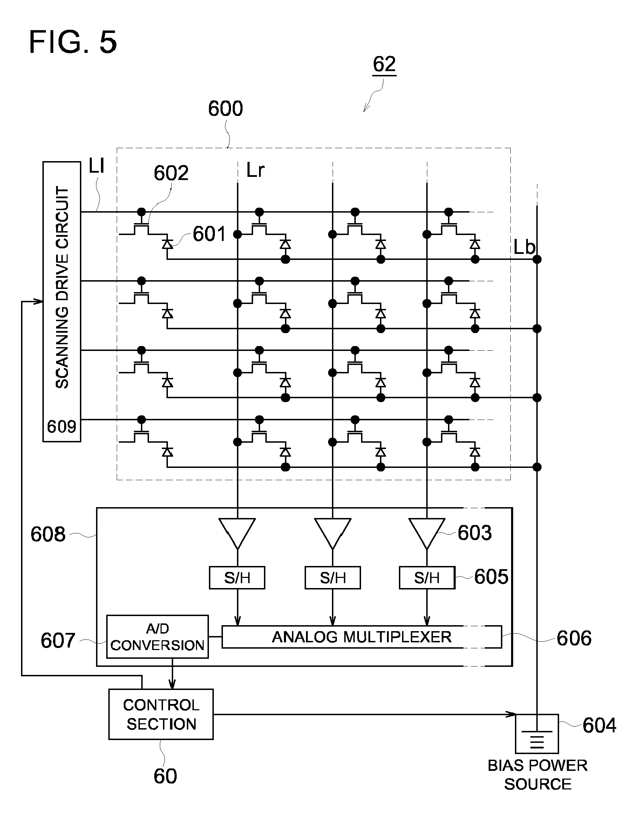

As shown in Fig. 4, each photoelectric conversion section is composed of a photodiode 601 and a thin film transistor (TFT) 602 for taking out electric energy stored in the photodiode 601 as an electric signal by switching. The taken-out electric signal is amplified by an amplifier 603 up to a level where it can be detected by a signal reading circuit 608 (refer to Fig. 5). Further, to the amplifier 603, a reset circuit, which is not illustrated, composed of the TFT 602 and a capacitor is connected and when the TFT 602 is switched, a reset operation of resetting the stored electric signal is performed. Further, the TFT 602 may be an inorganic semiconductor transistor used in a liquid crystal display or a transistor using an organic semiconductor. Further, the case where the photodiode 601 is used as a photoelectric conversion element is illustrated, however the photoelectric conversion element may be a solid-state imaging device other than the photodiode.

Fig. 5 is an equivalent circuit diagram of the signal detection section 600 having the photoelectric conversion sections arranged two-dimensionally and between the respective photoelectric conversion sections composing pixels, scanning lines L1 and signal lines Lr are arranged so as to intersect orthogonally. To each of the photodiodes 601 aforementioned, the TFT 602 is connected and one end of the side of each of the photodiodes 601 to which the TFT 602 is connected is connected to the signal line Lr. On the other hand, the other end of each of the photodiodes 601 is connected to one end of the neighboring photodiode 601 arranged in each row and then is connected to a bias power source 604 via a common bias line Lb. One end of the bias power source 604 is connected to a control section 60 and by an instruction from the control section 60, the photodiodes 601 are applied with a voltage via the bias line Lb.

The TFTs 602 arranged in each row are connected to the common scanning line L1 and the scanning lines L1 are connected to the control section 60 via a scanning drive circuit 609 for sending a pulse to each photoelectric conversion element. Similarly, the photodiodes 601 arranged in each line are connected to the common signal line Lr and then are connected to the signal reading circuit 608 controlled by the control section 60. In the signal reading circuit 608, in the ascending order of distance from the imaging panel 62, the amplifier 603, a sample hold circuit 605, an analog multiplexer 606, and the A-D converter 607 are arranged on each of the common signal lines Lr.

At time of signal reading, the scanning drive circuit 609 drives each of the TFTs 602 to the power supply state, thus the charge stored in the anode of the photodiode 601 is transmitted to the amplifier 603 as an electric signal. The electric signal is amplified by the amplifier 603 up to a level where it can be detected by the signal reading circuit 608. And, the voltage of the amplifier 603 is temporarily held by the sample hold circuit 605 and then is given to the analog multiplexer 606.

The analog multiplexer 606 converts the obtained voltage to a serial electric signal and transmits it to the A-D converter 607 and the A-D converter 607 converts the electric signal to digital data. In this way, radiation image data is prepared by the imaging panel 62.

In Fig. 3 again, the radiation image detecting apparatus 6 includes an indicator 63, a transmission button 64, a deletion button 65, an image storing section 66, a power source 67, and a charging terminal 69.

The image storing section 66 is composed of a rewritable memory such s a nonvolatile memory or a flash memory and stores the radiation image data outputted from the imaging panel 62. The image storing section 66 may be a built-in memory or a removable memory such as a memory card.

The indicator 63 is installed at one end of the surface of the frame 61 and displays the operation state of the radiation image detecting apparatus 6. For example, the indicator 63 can display and report the charging state of a charging battery 68 which will be described later and the transmission state of the radiation image data.

The transmission button 64 is installed in the neighborhood of the indicator 63 and when the transmission button 64 is pressed, transmission of the radiation image data stored in the image storing section 66 can be instructed. Further, the transmission button 64 has a built-in LED (light emitting diode) for emitting blue or red light and can display the operation state of the transmission button 64 by blinking, on, or off.

The deletion button 65 is installed in the neighborhood of the indicator 63 and when the deletion button 65 is pressed, deletion of the radiation image data stored in the image storing section 66 can be instructed. In this embodiment, the deletion button 65 is equivalent to a deletion instruction input section.

The power source 67 supplies power to a plurality of drive sections (the control section 60, imaging panel 62, indicator 63, input button 64, image storing section 66, etc.) composing the radiation image detecting apparatus 6. The power source 67 is composed of a spare battery 671 composed of, for example, a manganese cell, an alkaline cell, a lithium cell, or a nickel-cadmium cell and a chargeable battery 672 composed of, for example, a nicad cell, a nickel-hydrogen cell, a lithium-ion cell, a fuel cell, or a solar cell.

As mentioned above, the power source 67 has the spare battery 671 in addition to the chargeable battery 672, so that when the charging amount of the chargeable battery 672 is insufficient or during replacement of the chargeable 672, at least minimal power can be supplied to the radiation image detecting apparatus 6. Therefore, the image information stored in the image storing section 66 is not deleted by mistake and the apparatus does not enter the state where a signal from an external apparatus such as the console 7 cannot be received. Further, when the charging terminal 69 is connected to a cradle not illustrated, the chargeable battery 672 can be charged.

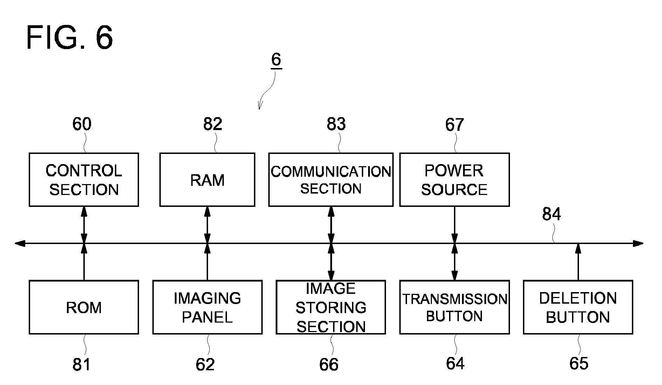

Fig. 6 is a block diagram showing the constitution of the essential section of the radiation image detecting apparatus 6. As shown in Fig. 6, the control system of the radiation image detecting apparatus 6 includes the control section 60, imaging panel 62, transmission button 64, deletion button 65, image storing section 66, power source section 67, ROM 81, RAM 82, and communication section 83 and the sections are connected to a bus 84. Further, among them, the imaging panel 62, transmission button 64, deletion button 65, image storing section 66, and power source section 67 are described above, so that the description therefor will be omitted here.

The control section 60 is composed of, for example, a CPU, reads the control program stored in the ROM 81, loads it into the work area formed in the RAM 82, and controls the sections of the radiation image detecting apparatus 6 according to the concerned control program. The control section 60 is equivalent to the deletion control device of the present invention.

The ROM 81 is composed of a nonvolatile semiconductor memory and stores the control program executed by the control section 60 and various programs.

The RAM 82, in various processes executed and controlled by the control section 60, forms a work area for temporarily storing various programs which are read from the ROM 81 and can be executed by the control section 60, input or output data, and parameters.

The communication section 83, by the wireless communication system such as a radio LAN, via the base station 5, communicates various information with the console 7. Further, the wireless communication mentioned above uses radio waves (space waves) and further includes light wireless communication using infrared rays and visible rays (laser or the like) and acoustic communication using acoustic waves or ultrasonic waves. In this embodiment, the communication section 83 and control section 80 can compose the image data transmission section (the second transmission section) and success signal receiving section (the second receiving section) of the present invention.

### (First embodiment)

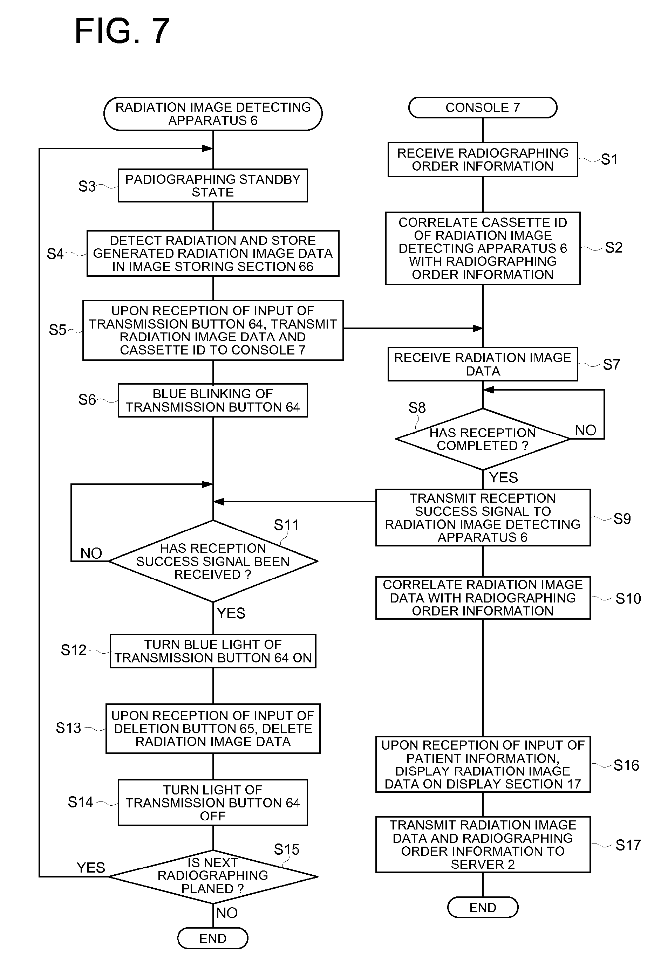

Fig. 7 is a flow chart for describing the operation of the radiation image radiographing system 1 relating to the first embodiment. By referring to the drawing, a series of operations for executing radiography for the patient 12, generating radiation image data, and transmitting the radiation image data to the console 7 by wireless communication will be explained. Further, in this embodiment, the image storing section 66 of the radiation image detecting apparatus 6 has one memory space for storing radiation image data only for one radiographing. Further, it is assumed that radiographing order information is inputted beforehand by a doctor or a receptionist and the concerned radiographing order information is stored in the external storage device of the server 2.

Firstly, when executing radiation image radiography, the console 7 receives the radiographing order information stored in the server 2 (Step S1) and stores the concerned radiographing order information in the storing section 21. An operator such as a doctor or a radiology technician confirms the radiographing information included in the radiographing order information, then selects the radiation image detecting apparatus 6 suited to radiography from a plurality of radiation image detecting apparatuses, and inputs the cassette ID assigned beforehand to the concerned radiation image detecting apparatus 6 by using the input operation section 18. The console 7, upon receipt of input of the cassette ID, correlates the cassette ID with the radiographing order information (Step S2) and stores all of them in the storing section 21. And, the console 7 correlates the console ID assigned beforehand to the console 7 with the cassette ID and radiographing order information and transmits it to the server 2, and transmits the radiographing information included in the radiographing order information to the radiographing operation apparatus 4. Further, the console 7 transmits the console ID assigned to the console 7 to the radiation image detecting apparatus 6.

The radiation image detecting apparatus 6 selected by the operator is mounted at the mounting port of the detecting apparatus installed under the bed 11 and is put into the radiographing standby state (Step S3). Further, the operator, to set the part to be radiographed according to the radiographing information at the suitable position for the radiation image radiographing apparatus 3, loads the patient 12 on the bed 11. And, on the basis of the radiographing information received from the console 7, the radiation image detecting apparatus 6 controls the radiographing operation apparatus 4 and radiation is emitted from the radiation image radiographing apparatus 3 on the basis of the concerned control information. The radiation image detecting apparatus 6 detects the radiation which is emitted from the radiation image radiographing apparatus 3 and has passed through the patient 12, generates radiation image data in the aforementioned process, and stores it in the image storing section 66 (Step S4). By doing this, the image storing section 66 stores the radiation image data of one radiographing and is kept in the state where radiation image data beyond it cannot be stored. Here, radiation image data of one radiographing means radiation image data for a set of radiographing generated when a radiation image necessary to diagnose a patient is captured, and for example, when one patient is radiographed only once, one image corresponds to it, and when one patient is continuously radiographed at a plurality of angles, a plurality of images radiographed at a plurality of angles correspond to it, and when a plurality of patients are continuously radiographed in the group medical examination, images of the plurality of patients correspond to it. Further, when moving images are captured, a plurality of images of predetermined frames from radiographing start to radiographing end correspond to it.

When one radiographing ends, the operator takes out the radiation image detecting apparatus 6 from the mounting port of the detecting apparatus of the bed 11 and presses the transmission button 64 of the radiation image detecting apparatus 6. The radiation image detecting apparatus 6, upon receipt of input of the transmission button 64, permits the radiation image data stored in the image storing section 66 to be accompanied with the cassette ID and transmits it from the communication section 83 by wireless communication via the base station 5 (Step S5). At this time, the radiation image data and cassette ID, on the basis of the console ID, are transmitted to the console 7. And, the transmission button 64 blinks in blue (Step S5), indicating that the radiation image data is in transmission.

The console 7 receives the radiation image data (and cassette ID) transmitted from the radiation image detecting apparatus 6 (Step S7) and when the reception of the radiation image data is completed (Yes at Step S8), transmits a reception success signal to the radiation image detecting apparatus 6 via the base station 5 (Step S9). Further, the console 7 performs the image processing such as the A-D conversion, normalization processing, and gradation processing for the radiation image data received, then correlates the radiation image data with the radiographing order information on the basis of the cassette ID accompanied with the radiation image data (Step S10), and stores them in the image storing section 21.

The radiation image detecting apparatus 6, upon receipt of the reception success signal from the console 7 (Yes at Step S11), turns on the transmission button 64 in blue (Step S12) to display completion of the data transmission. The operator confirms that the transmission button 64 is turned on in blue and presses the deletion button 65. The radiation image detecting apparatus 6, upon receipt of input of the deletion button 65, deletes the radiation image data stored in the image storing section 66 (Step S13). And, the radiation image detecting apparatus 6 turns off the transmission button 64 (Step S14). When the next radiography is planned (Yes at Step S15), the radiation image detecting apparatus 6 repeats Steps S3 to S14 aforementioned and when the radiography ends (No at Step S15), the process by the radiation image detecting apparatus 6 is finished.

When the radiography ends, the operator operates the input operation section 18 of the console 7 and inputs the patent information such as the patient name and patient ID. The console 7, upon receipt of input of the patient information by the input operation section 18, displays the radiation image data correlated to the concerned patient information on the display section 17 (Step S16). And, the operator confirms the radiation image data displayed, and when there is no problem, transmits the radiation image data and radiographing order information to the server 2 (Step S17), and the process of the console 7 is finished.

As mentioned above, this embodiment is structured so that the console 7 completes reception of the radiation image data and then transmits the reception success signal, and the radiation image detecting apparatus 6 receives the concerned reception success signal and then deletes the radiation image data. Therefore, when a communication error occurs during transmission of the radiation image data, thus the console 7 cannot receive the radiation image data, the reception success signal is not transmitted from the console 7, so that the radiation image detecting apparatus 6 does not delete the radiation image data. Therefore, even if a communication error occurs, the radiation image detecting apparatus 6 can retransmit the radiation image data to the console 7, so that there is no need to execute re-radiography and the radiographing operation can be performed smoothly. Further, the patient will not be exposed again to the radiation.

Further, as mentioned above, the radiation image detecting apparatus 6 is composed of an FPD and the radiation image detecting apparatus 6 and console 7 can transmit and receive the radiation image data by wireless communication, so that the immediacy of image data output of the radiation image detecting apparatus 6 can be used at a maximum. Further, even in continuous radiography, there is no need to increase the capacity of the image storing section 66 of the radiation image detecting apparatus 6, so that the radiation image detecting apparatus 6 can be made smaller.

Further, the embodiment aforementioned is structured so that the radiation image detecting apparatus 6, upon receipt of the reception success signal from the console 7, judges that the reception by the console 7 succeeds and moves to the deletion operation, however it may be structured so that when the console 7 fails in reception, it transmits a reception failure signal without transmitting the reception success signal. In this case, when the radiation image detecting apparatus 6 does not receive the reception failure signal within a predetermined period of time, it judges that the reception succeeds and it can move to the deletion operation.

Further, the embodiment is structured so that the radiation image detecting apparatus 6, upon receipt of input from the transmission button 64, transmits the radiation image data to the console 7 and upon receipt of input from the deletion button 65, deletes the radiation image data. However, it may be structured so as to input a transmission instruction and a deletion instruction by the input operation section 18 of the console 7, thereby transmit the transmission instruction and deletion instruction to the radiation image detecting apparatus 6 by wireless communication to transmit or delete the radiation image data.

Further, it may be structured so that the radiation image data, when the radiation image detecting apparatus 6 receives the reception success signal, is deleted automatically. By use of such a constitution, the operator does not need to press the deletion button 65, so that the radiographing operation can be performed efficiently.

Further, the embodiment is structured so that at Step S2, the console ID of the console 7 is transmitted to the radiation image detecting apparatus 6 and at Step S5, on the basis of the concerned console ID, the radiation image detecting apparatus 6 transmits the radiation image data to the console 7. However, the embodiment may be structured so as to ask the server 2 about the console 7 of the transmission destination at Step S5 without transmitting beforehand the console ID of the console 7 to the radiation image detecting apparatus 6. Concretely, the embodiment is structured so that, at Step S2, the correlation of the console ID of the console 7 to the cassette ID of the radiation image detecting apparatus 6 is stored in the server 2 and when the transmission destination is inquired from the radiation image detecting apparatus 6 at Step S5, the server 2 selects the console ID correlated to the cassette ID of the radiation image detecting apparatus 6 and the concerned console ID is transmitted to the radiation image detecting apparatus 6.

Further, the embodiment may be structured so that the radiation image detecting apparatus 6 generates reduced image data (subsampled data) smaller in information amount than the radiation image data, transmits the reduced image data before transmitting the radiation image data to the console 7, so as to confirm the image. By use of such a constitution, on the basis of the reduced image data at the console 7, the radiographing conditions (the position of the patient, radiation dosage, etc.) are confirmed and when reradiographing is necessary, reradiographing can be executed before receiving the radiation image data, so that the radiographing operation can be performed efficiently.

### (Modification)

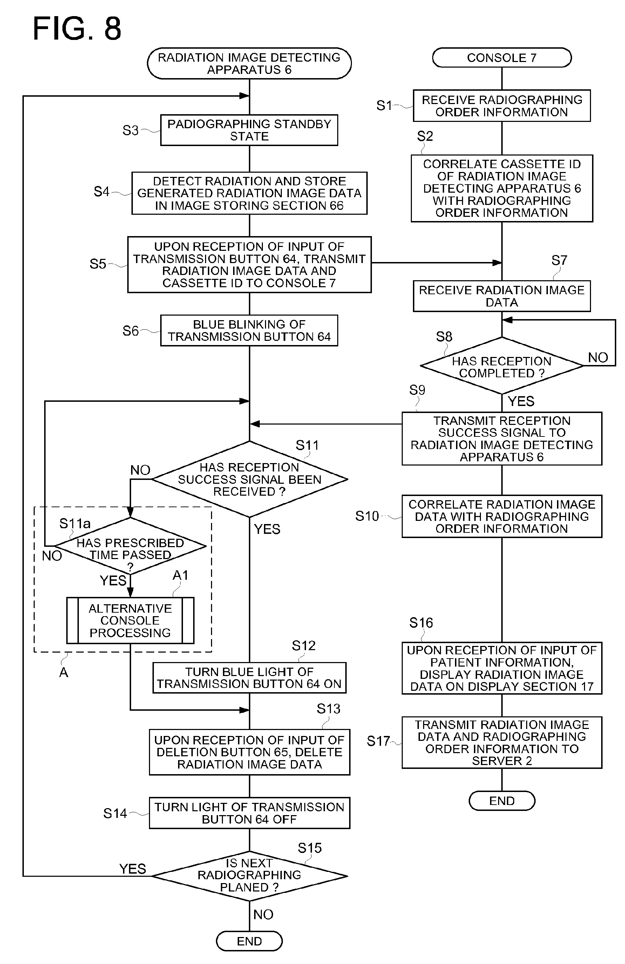

Fig. 8 is a flow chart when the operation of the radiation image radiographing system 1 relating to the first embodiment shown in Fig. 7 is partially modified. That is, this modification is structured so that when the radiation image detecting apparatus 6 does not receive the reception success signal from the console 7 within a predetermined period of time, the radiation image data is retransmitted to an alternative console different from the console 7. Further, Fig. 8 is different in a dashed line portion A from Fig. 7, so that here, the dashed line portion A will be explained mainly and the explanation will be omitted for the duplicated processes. Further, the alternative console is a console to which the console ID next to the console ID assigned to the console 7 is assigned.

At Step S11 shown in Fig. 8, when the radiation image detecting apparatus 6 does not receive the reception success signal from the console 7 even after a lapse of a predetermined period of time (Yes at Step S11a), it conducts the alternative console processing (Step A1).

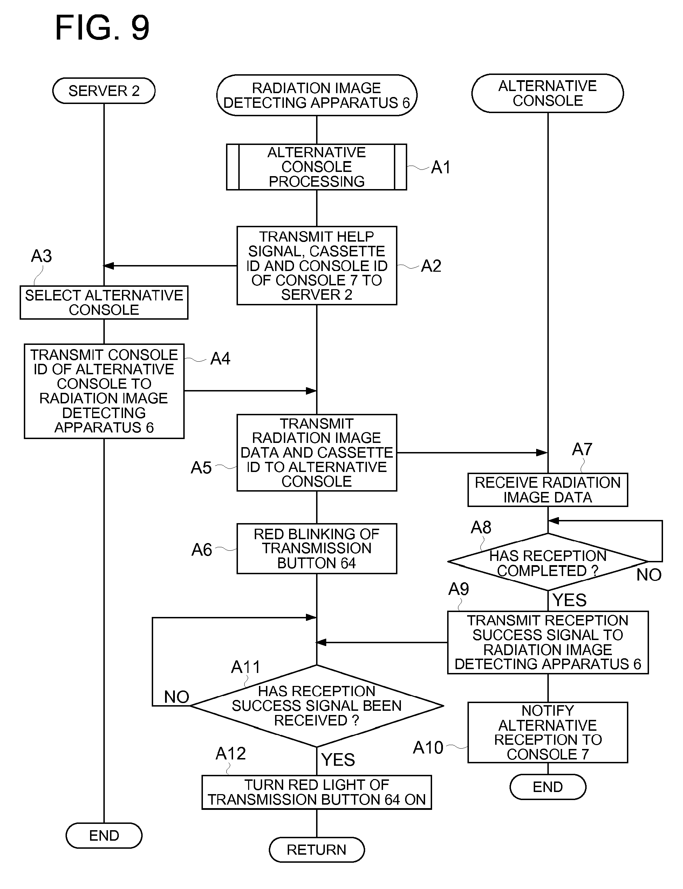

Fig. 9 is a flow chart for explaining the process of the alternative console of the radiation image detecting apparatus 6 in the radiation image radiographing system 1.

Firstly, when the radiation image detecting apparatus 6 does not receive the reception success signal from the console 7 even after a lapse of a predetermined period of time, to the server 2, it transmits a help signal indicating that it does not receive the reception success signal and transmits also the cassette ID of the radiation image detecting apparatus 6 and the console ID of the console 7 accompanied with the held signal (Step A2). The server 2, upon receipt of the help signal from the radiation image detecting apparatus 6, retrieves the alternative console assigned with the ID next to the console ID of the console 7 from the external storing device and selects the concerned alternative console as a retransmission destination (Step A3). And, the server 2 transmits the console ID of the alternative console to the radiation image detecting apparatus 6 (Step A4).

The radiation image detecting apparatus 6, upon receipt of the console ID of the alternative console from the server 2, retransmits the radiation image data (and cassette ID) having been transmitted to the console 7 to the alternative console (Step A5). At this time, the transmission button 64 blinks in red (Step A6), indicating that the radiation image data is in transmission to the alternative console.

The alternative console receives the radiation image data transmitted from the radiation image detecting apparatus 6 (Step A6) and when the reception of the radiation image data is completed (Yes at Step A8), transmits the reception success signal to the radiation image detecting apparatus 6 (Step A9). Further, at this time, the alternative console notifies the console 7 of alternative reception (Step A10).

The radiation image detecting apparatus 6, upon receipt of the reception success signal from the alternative console (Yes at Step A11), turns on the transmission button 64 in red (Step A12), returns to Fig. 9, and continues the process.

As mentioned above, when the radiation image detecting apparatus 6 does not receive a reception completion signal from the console 7 within a predetermined period of time, it retransmits the radiation image data to an alternative console different from the console 7. Therefore, for example, even if the console 7 is in the communication impossible state due to a fault, the radiation image data is transmitted to the alternative console and stored in it, so that the radiographing operation can be performed smoothly.

Further, in the above explanation, the console assigned with the console ID next to the console ID assigned to the console 7 is selected as an alternative console. Generally, the consoles whose console IDs are next to each other are often installed close to each other, so that by selecting such a console as an alternative console, the radiation image data is transmitted to the close alternative console. Therefore, even if the alternative console processing is performed due to impossible communication, the operator can quickly respond to it. Further, needless to say, the console whose console ID is next to the consol ID may not be selected as an alternative console.

Further, when performing the alternative console processing, the display color of the transmission button 64 is set at a different color (red) from the color in the ordinary state, so that the operator can easily learn that the radiation image data is transmitted to the alternative console.

Further, the modification is structured so that when the radiation image detecting apparatus 6 does not receive the reception success signal even after a lapse of a predetermined period of time, it transmits a help signal to the server 2 and upon receipt of the help signal, the server 2 selects the alternative console. However, the present invention is not limited to it and it is possible, for example, upon receipt of the concerned help signal, to transmit a list of consoles to the radiation image detecting apparatus 6 from the server 2 and to select an alternative console from the list of consoles by the radiation image detecting apparatus 6.

### (Second embodiment)

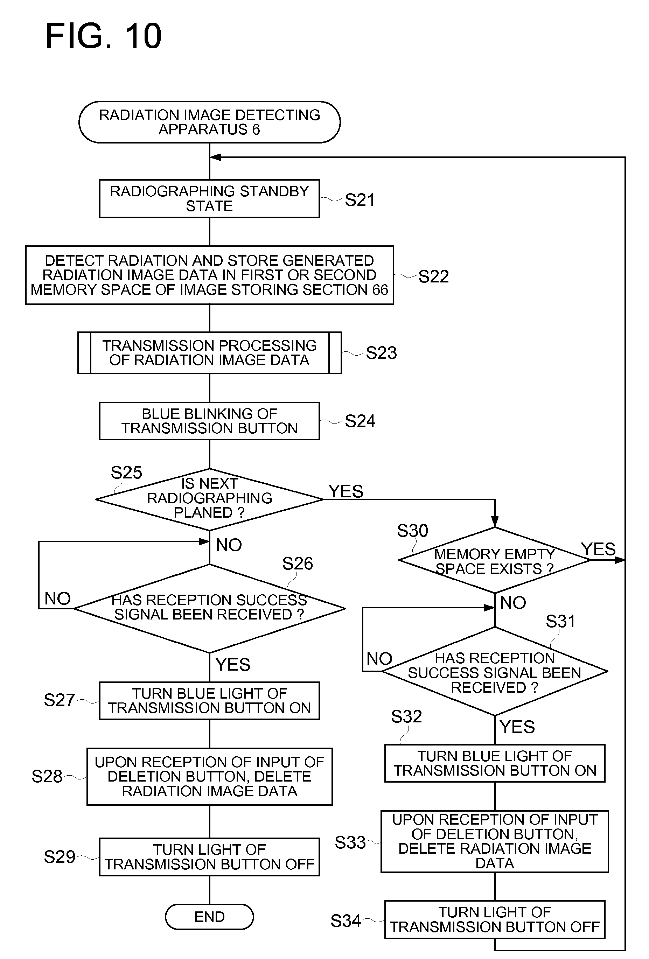

Fig. 10 is a flow chart for describing the operation of the radiation image radiographing system 1 relating to the second embodiment. In the first embodiment aforementioned, the image storing section 66 of the radiation image detecting apparatus 6 has a memory space for storing radiation image data of only one radiographing, while in this embodiment, the image storing section 66 has a first memory space and a second memory space each of which can store radiation image data of one radiographing, thereby can store simultaneously radiation image data of two sets of radiographing. Further, to control independently the first memory space and second memory space, the image storing section 66 includes a first transmission button and a first deletion button and a second transmission button and a second deletion button (they are not illustrate) in response to the respective memory spaces. Further, the operation of the console 7 is the same as that of the first embodiment, so that in Fig. 8, the operation of the radiation image detecting apparatus 6 will be explained mainly.

Firstly, when executing radiation image radiography, as explained in the first embodiment, the console 7 receives the radiographing order information stored in the server 2 and stores the concerned radiographing order information in the storing section 21. The operator confirms the radiographing information included in the radiographing order information, then selects the radiation image detecting apparatus 6 suited to radiography from a plurality of radiation image detecting apparatuses, and inputs the cassette ID assigned to the concerned radiation image detecting apparatus 6 by the input operation section 18. The console 7, upon receipt of input of the cassette ID, correlates the cassette ID with the radiographing order information and stores all of them in the storing section 21. And, the console 7 correlates the console ID assigned to the console 7 with the cassette ID and radiographing order information and transmits it to the server 2, and transmits the radiographing information included in the radiographing order information to the radiographing operation apparatus 4 (refer to Steps S1 and S2 shown in Fig. 7). Further, the console 7 transmits the console ID thereof to the radiation image detecting apparatus 6.

In Fig. 8, the radiation image detecting apparatus 6 selected by the operator is mounted at the mounting port of the detecting apparatus installed under the bed 11 and is put into the radiographing standby state (Step S21). Further, the operator, to set the part to be radiographed according to the radiographing information at the suitable position to the radiation image radiographing apparatus 3, loads the patient 12 on the bed 11. And, on the basis of the radiographing information received from the console 7, the operator controls the radiographing operation apparatus 4 and radiation is emitted from the radiation image radiographing apparatus 3 on the basis of the control information. The radiation image detecting apparatus 6 detects the radiation which is emitted from the radiation image radiographing apparatus 3 and has passed through the patient 12, generates radiation image data according to the aforementioned process, and stores it in the first memory space or second memory space of the image storing section 66 (Step S22). In the first radiography, the first memory space and second memory space are in the empty state and here, firstly, the radiation image data is stored in the first memory space.

When one radiographing ends, the operator takes out the radiation image detecting apparatus 6 from the mounting port of the detecting apparatus of the bed 11 and presses the first transmission button installed in the radiation image detecting apparatus 6. The radiation image detecting apparatus 6, upon receipt of input of the first transmission button, permits the radiation image data stored in the first memory space of the image storing section 66 to be accompanied with the cassette ID and performs the transmission process to the console 7 by wireless communication via the base station 5 (Step S23). Further, at this time, the first transmission button blinks in blue (Step S24), indicating that the radiation image data is in transmission.

Thereafter, when the next radiographing is not planned (No at Step S25), the radiation image detecting apparatus 6 waits for reception of the reception success signal from the console 7. And, when receiving the reception success signal from the console 7 (Yes at Step S26), the radiation image detecting apparatus 6 turns on the first transmission button in blue (Step S27) to indicate that the data transmission is completed. The operator confirms that the first transmission button is turned on in blue and presses the first deletion button. The radiation image detecting apparatus 6 receives input of the first deletion button and deletes the radiation image data stored in the first memory space of the image storing section 66 (Step S28). And, the radiation image detecting apparatus 6 turns off the first transmission button (Step S29) and the process is finished.

When executing the next radiographing (Yes at Step S25), whether the first and second memory spaces of the image storing section 66 are empty or not is judged. For example, when the radiation image data is stored only in the first memory space and the second memory space is empty (Yes at Step S30), back to Step S21, the radiography is executed again, and the generated radiation image data is stored in the second memory space. As mentioned above, at this step, the process of transmitting the radiation image data of the first radiographing stored in the first memory space to the console 7 and the process of generating the radiation image data of the second radiographing and storing it in the second memory space can be performed in parallel. And, when the next radiographing is not planned, the radiation image data stored in the first and second memory spaces are deleted and the process is finished (Steps S26 to S29).

On the other hand, when executing the next radiographing (Yes at Step S25), and when the radiation image data is stored in both first and second memory spaces of the image storing section 66, and the memory spaces are not empty (No at Step S30), in order to delete the radiation image data, the radiation image detecting apparatus 6 is kept in the standby state until it receives the reception success signal from the console 7. Generally, the radiation image data of the first memory space, which is generated earlier, is firstly transmitted to the console 7, so that the radiation image detecting apparatus 6 receives firstly the reception success signal of the radiation image data stored in the first memory space.

When receiving the reception completion signal from the console 7 (Yes at Step S31), the radiation image detecting apparatus 6 turns on the first transmission button in blue (Step S32) to indicate that the data transmission is completed. The operator confirms that the first transmission button is turned on in blue and presses the first deletion button. The radiation image detecting apparatus 6 receives input of the first deletion button and deletes the radiation image data stored in the first memory space of the image storing section 66 (Step S33). And, the radiation image detecting apparatus 6 turns off the first transmission button (Step S34), then returns to Step S21, performs the radiographing again, and stores the radiation image data in the first memory space.

As mentioned above, in this embodiment, the image storing section 66 of the radiation image detecting apparatus 6 has a capacity of storing radiation image data of two sets of radiographing and if either of the memory spaces is empty when executing the next radiographing, the transmission operation of the radiation image data and the operation of the next radiographing can be performed in parallel. Therefore, the memory capacity of the image storing section 66 is suppressed inasmuch as it is possible, and the size of the radiation image detecting apparatus 6 is reduced, while the operation can be performed efficiently.

Further, in the above explanation, efficiency improvement takes priority, so that if the first memory space or second memory space is empty, the next radiographing operation is executed immediately and the transmission operation of the radiation image data and the radiographing operation are structured so as to be performed in parallel inasmuch as is possible. However, it is possible, for example, to execute two sets of radiographing, to store the radiation image data in the first memory space and second memory space, and to transmit all the radiation image data of two sets of radiographing to the console 7 or after deleting both data in the first memory space and second memory space to move to the next radiographing operation. These processes can be changed properly according to the radiographing flow of the operator or to apparatus constitution.

Further, each of the first memory space and second memory space has a memory capacity of storing only radiation image data of one radiographing, however for example, needless to say, each of both memory spaces can have a memory capacity of storing radiation image data of a plurality of sets of radiographing.

Further, needless to say, the alternative console processing aforementioned can be applied to the second embodiment.

According to the present invention, the radiation image detecting apparatus deletes the radiation image data after receiving the reception success signal, so that even if a communication error occurs, the radiation image detecting apparatus can retransmit the radiation image data to the console. Therefore, there is no need to execute reradiographing, and the radiation image detecting apparatus can be miniaturized inasmuch as is possible, and the operation of radiography can be performed smoothly.

## Claims

1. A radiation image radiographing system connected to a network, the radiation image radiographing system comprising:
a radiation image detecting apparatus for generating radiation image data by detecting a radiation having passed through a subject, the radiation image detecting apparatus being portable and capable of transmitting the generated radiation image data; and
a console for being used to register radiographing order information relating to radiographing and for correlating the radiographing order information with the radiation image detecting apparatus, the console comprising:
a first receiving section for receiving the radiation image data transmitted from the radiation image detecting apparatus through wireless communication; and
a first transmitting section for transmitting a reception success signal to the radiation image detecting apparatus after the radiation image data is successfully received,
wherein the radiation image detecting apparatus comprising:
an image storing section for storing the generated radiation image data for at least one set of radiographing;
a second transmitting section for transmitting, to the console, the radiation image data stored in the image storing section through wireless communication;
a second receiving section for receiving the reception success signal transmitted from the first transmitting section; and
a deletion control device for controlling so as to delete, from the image storing section, the radiation image data transmitted from the second transmitting section if the reception success signal is received by the second receiving section after the second transmitting section transmits the radiation image data.

2. The radiation image radiographing system of claim 1,
wherein the image storing section has a plurality of memory spaces in which the deletion control device can control to delete the radiation image data respectively.

3. The radiation image radiographing system of claim 1 or 2, further comprising,
a deletion instruction input section for inputting a deletion instruction of the radiation image data,
wherein the deletion control device deletes, from the image storing section, the radiation image data transmitted from the second transmitting section based on the deletion instruction inputted by the deletion instruction input section.

4. The radiation image radiographing system of any one of claims 1 - 3,
wherein the console has a correlating device for correlating the radiation image detecting apparatus to be used for radiographing with the radiographing order information relating to the radiographing.

5. The radiation image radiographing system of claim 4,
wherein the second transmitting section transmits the radiation image data to the console correlated by the correlating device.

6. The radiation image radiographing system of claim 5, comprising:
a plurality of consoles; and
a selecting device for selecting an alternative console different from the correlated console as a transmitting destination of the radiation image data if the second receiving section does not receive the reception success signal within a predetermined time after the second transmitting section transmits the radiation image data.

7. The radiation image radiographing system of claim 6,
wherein an ID is allocated to each of the plurality of consoles and the selecting device selects a console as the alternative console, an ID allocated to the console being next to an ID allocated to the correlated console.

8. The radiation image radiographing system of claim 6 or 7,
wherein the second transmitting section transmits the radiation image data to the alternative console selected by the selecting device.

9. The radiation image radiographing system of any one of claims 6 - 8, further comprising:
a server for storing the ID allocated to each of the plurality of consoles, the server having the selecting device.

10. A radiation image detecting apparatus being portable and capable of transmitting radiation image data generated by detecting a radiation having passed through a subject, to a console in which a radiographing order information relating to radiographing has been registered, the radiation image detecting apparatus comprising:
an imaging panel for generating the radiation image data by detecting a radiation having passed through a subject;
an image storing section for storing the radiation image data which is generated by the imaging panel for at least one set of radiographing;
an image data transmitting section for transmitting the radiation image data stored in the image storing section to the console through wireless communication;
a success signal receiving section for receiving a reception success signal indicating that the radiation image data has been successfully received by the console; and
a deletion control device for controlling to delete, from the image storing section, the radiation image data transmitted from the image data transmitting section if the reception success signal is received by the success signal receiving section after the image data transmitting section transmits the radiation image data.

11. The radiation image detecting apparatus of claim 10,
wherein the image storing section has a plurality of memory spaces in which the deletion control device can control to delete the radiation image data respectively.

12. The radiation image detecting apparatus of claim 10 or 11, further comprising,
a deletion instruction input section for inputting a deletion instruction of the radiation image data,
wherein the deletion control device deletes, from the image storing section, the radiation image data transmitted from the image data transmitting section based on the deletion instruction inputted by the deletion instruction input section.

13. The radiation image detecting apparatus of any one of claims 10 - 12,
wherein the image data transmitting section transmits the radiation image data to an alternative console if the success signal receiving section does not receive the reception success signal within a predetermined time after the image data transmitting section transmits the radiation image data.
